Europäisches Patentamt

European Patent Office

Office européen des brevets

(11) Publication number: **0 358 168 B1**

# EUROPEAN PATENT SPECIFICATION

(45) Date of publication of patent specification: **30.11.94**   (51) Int. Cl.5: **G01N 27/406**

(21) Application number: **89116377.6**

(22) Date of filing: **05.09.89**

(54) Method, apparatus and probe for measuring the activity of a solute element in molten metal.

(30) Priority: **07.09.88 JP 224336/88**
**14.09.88 JP 230984/88**

(43) Date of publication of application:
**14.03.90 Bulletin 90/11**

(45) Publication of the grant of the patent:
**30.11.94 Bulletin 94/48**

(84) Designated Contracting States:
**BE DE FR GB**

(56) References cited:
**EP-A- 0 059 222**
**EP-A- 0 208 072**
**EP-A- 0 295 112**
**US-A- 3 661 725**
**US-A- 4 830 727**

**PATENT ABSTRACTS OF JAPAN, vol. 10, no. 341 (P-517)[2397], 18th November 1986;& JP-A-61 142 455**

**PATENT ABSTRACTS OF JAPAN, vol. 11, no. 112 (P-565)[2559], 9th April 1987;& JP-A-61 260 156**

**PATENT ABSTRACTS OF JAPAN, vol. 11, no. 112 (P-565)[2559], 9th April 1987;& JP-A-61 260 157**

(73) Proprietor: **Sasabe, Minoru**
**8-22-20-501, Konakadai**
**Chiba-shi Chiba-ken (JP)**

Proprietor: **NKK CORPORATION**
**1-2 Marunouchi 1-chome,**
**Chiyoda-ku**
**Tokyo (JP)**

Proprietor: **Osaka Sanso Kogyo Limited**
**1-14, Miyahara 4-chome**
**Yodogawa-ku Osaka (JP)**

(72) Inventor: **Sasabe, Minoru**
**8-22-20-501, Konakadai**
**Chiba-shi, Chiba-ken (JP)**
Inventor: **Hasegawa, Teruyuki c/o Patent & License Dept.,**
**NKK CORPORATION, 1-2, 1-chome, Marunouchi, Chiyoda-ku, Tokyo (JP)**
Inventor: **Ishikawa, Hiroaki c/o Patent & License Dept.,**
**NKK CORPORATION, 1-2, 1-chome, Marunouchi, Chiyoda-ku, Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

Inventor: **Kawai, Yoshihiko c/o Patent & License Dept.,**
**NKK CORPORATION, 1-2, 1-chome,**
**Marunouchi, Chiyoda-ku, Tokyo (JP)**
Inventor: **Furuta, Chikayoshi c/o Osaka Sanso Kogyo Ltd.,**
**1-14, Miyahara 4-chome,**
**Yodogawa-ku, Osaka (JP)**
Inventor: **Nagatsuka, Toshio c/o Osaka Sanso Kogyo Ltd.,**
**1-14, Miyahara 4-chome**
**Yodogawa-ku, Osaka (JP)**
Inventor: **Matsushige, Haruhiko c/o Osaka Sanso Kogyo Ltd.,**
**1-14, Miyahara 4-chome**
**Yodogawa-ku, Osaka (JP)**

(74) Representative: **Sajda, Wolf E., Dipl.-Phys. et al**
**MEISSNER, BOLTE & PARTNER**
**Widenmayerstrasse 48**
**D-80538 München (DE)**

2

**Description**

The present invention relates to a probe for measuring the activity of a solute element in molten metal, such as molten steel, especially the activity of Mn, Si, Cr, or P, and also to an apparatus and a method for measuring the activity of the solute element in the molten metal by the use of the probe.

Modern metal products are diverse and must be of high quality. It is essential, therefore, properly to control solute elements in molten metal during a refining process. Thus, various analytical measuring means have been proposed to grasp the progress of refining reactions.

Instrumental analysis is conventionally known as a method for determining the contents of solute elements and the like. According to this method, however it requires a lot of time to obtain analytical results after sampling. In converter operation in the steel industry, in particular, it is necessary quickly to grasp the decarburization rate and the reduction rate of manganese ore. Therefore, the instrumental analysis method cannot be suitably applied to the converter operation. Accordingly, a sub-lance system has started to be practically used which can measure the activity of a solute element in molten steel in a short period of time, without extracting samples during a converter refining process.

In the sub-lance system, a probe having a solid electrolyte is attached to the distal end of a sub-lance, and is immersed in molten steel to measure oxygen electromotive force. In general, the molten steel temperature and carbon concentration are measured simultaneously with the electromotive force.

Disclosed in Published Unexamined Japanese Patent Application Nos. 61-142455 and 63-309849 are methods in which the concentration or activity of a solute element or the like in molten metal is measured by means of a probe. These methods are activity measuring methods for solute elements which utilize the principle of an oxygen concentration electrochemical cell. According to these methods, a coating layer is formed on the surface of a solid electrolyte of the probe. The coating layer is composed of an oxide of a solute element to be measured or a composite oxide of the same and another oxide. If the probe with this construction is immersed in the molten metal, equilibrium is produced between the solute element and the oxide thereof. The oxygen partial pressure caused by the reaction of equilibrium is measured, and the concentration or activity of the solute element can be obtained on the basis of the measured partial pressure. In the aforesaid prior art methods, the electromotive force E2 of the solute element to be measured and molten metal temperature T are actually measured. Based on the measured values, the activity $a_{M1}$ of the solute element to be measured is obtained according to a specific equation $a_{M1} = f(E2, T)$, or is obtained according to $a_{M1} = f(a_{O2}, T)$ after utilizing a relation $a_{O2} = f(E2, T)$ for the oxygen activity in a local equilibrated zone.

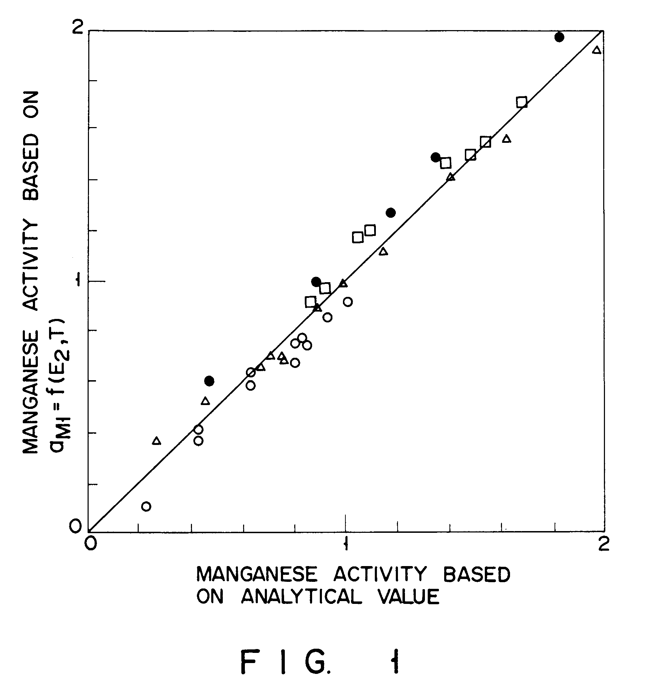

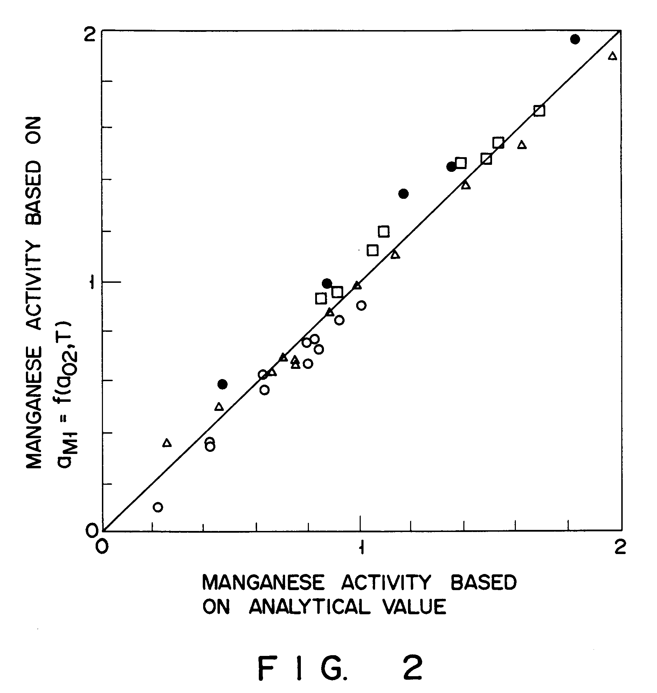

If Mn is measured in a converter, however, the measurement results obtained according to the conventional activity measuring methods described above are subject to substantial variations, as shown in Figs. 1 and 2. Accordingly, the content of a solute element and the like in molten steel cannot be measured with high accuracy, and the ingredients of the molten steel are subject to variation. Thus, the conventional methods cannot be suitably applied to refining of high-quality steel.

Disclosed in U.S. Pat. No. 4,830,727 is a probe and a concentration measuring method capable of highly accurately measuring the concentrations of solute elements in molten iron. This probe singly incorporates various sensor bodies, which are arranged corresponding individually to various solute elements. With this arrangement, the activities (actually, electromotive forces) of a plurality of solute elements can be simultaneously measured.

The use of the probe of this type is intended considerably to improve the concentration measuring accuracy. Thus, by converting the activity of a solute element to be measured into a concentration, the activities of other constituent elements can be taken into consideration at the same time.

Also in this measuring method, however, the activity of the element to be measured considerably varies depending on the measuring conditions. It was found that such variation is attributable to the oxygen activity $a_{O1}$ of a bulk, which had not been taken into consideration with respect to the conventional probes.

It was also revealed that the bulk oxygen activity $a_{O1}$ greatly influences the variation of the measurement under the following circumstances.

(1) The bulk oxygen activity $a_{O1}$ is substantially equal to the activity $a_{M1}$ of the solute element to be measured. ("Substantially equal" means that the two values are of the same order or different by only one figure.)

(2) The bulk oxygen activity $a_{O1}$ varies considerably or over the range of a substantially tenfold scale.

In these cases, the bulk oxygen activity or electromotive force must be considered in converting a measured electromotive force into an activity.

The measuring accuracy can be further improved by using the activity calculated as aforesaid in combination with the concentration measuring method described in U.S. Pat. No. 4,830,727.

In the probe described above, moreover, one sensor body is required for each ingredient of the solute element, so that the probe diameter must be increased in proportion to the number of elements to be measured. If the probe diameter is too large, it takes a lot of time to attain equilibrium after the probe is immersed in the molten metal, that is, it is difficult to measure the activity quickly and securely. Accordingly, probes for simultaneously measuring a multitude of elements are expected to be reduced in diameter.

An object of the present invention is to provide a method for measuring the activity of an element in molten metal, whereby the activities of Mn, Si, Cr, P, etc. in the molten metal can be simultaneously measured with high accuracy. And, an object of the present invention is to incorporate the oxygen activity $a_O$ or carbon concentration [C] of a bulk into a calculation formula in estimating the activities of the aforesaid elements.

Another object of the present invention is to collectively measure a plurality of solute elements to be measured. By this, the diameter of the probe can be reduced.

According to an aspect of the present invention, there is provided a method for measuring the activity of a solute element in molten metal, which comprises steps of: measuring the electromotive force E1 corresponding to oxygen content in bulk of the molten metal by means of a solid electrolyte having oxygen ion conductivity and measuring the temperature T of the molten metal; measuring the electromotive force E2 corresponding to content of the solute element in the molten metal by means of a solid electrolyte having oxygen ion conductivity and coated with an oxide containing the solute element to be measured; and obtaining the activity $a_M$ ofthe solute element to be measured on the basis of the measured electromotive forces E1 and E2 and the measured temperature T.

Thus, the electromotive forces E1 and E2 may be measured separately by means of different sensor bodies, or otherwise, the electromotive force E2 of balance portion A and the electromotive force E1 of balance portion B may be measured together by means of one sensor body.

According to still another aspect of the present invention, there is provided a method for measuring the activity of a solute element in molten metal, which comprises steps of: measuring the temperature T and solidifying temperature Ts of the molten metal; obtaining the carbon concentration [C] of the molten metal on the basis of the solidifying temperature Ts; measuring the electromotive force E2 of the solute element to be measured in the molten metal by means of a solid electrolyte having oxygen ion conductivity and coated with an oxide containing the solute element to be measured; and obtaining the activity $a_M$ of the solute element to be measured on the basis of the measured electromotive force E2, the carbon concentration [C], and the measured temperature T.

When measuring the electromotive forces E1 and E2 by means of different sensor bodies, the sensor body as the means for measuring the oxygen electromotive force E1 may be any oxygen sensor which is generally used for molten metal. Meanwhile, an body coated with, e.g., manganese oxide MnO may be used as the sensor body for measuring the electromotive force E2 of the solute element to be measured.

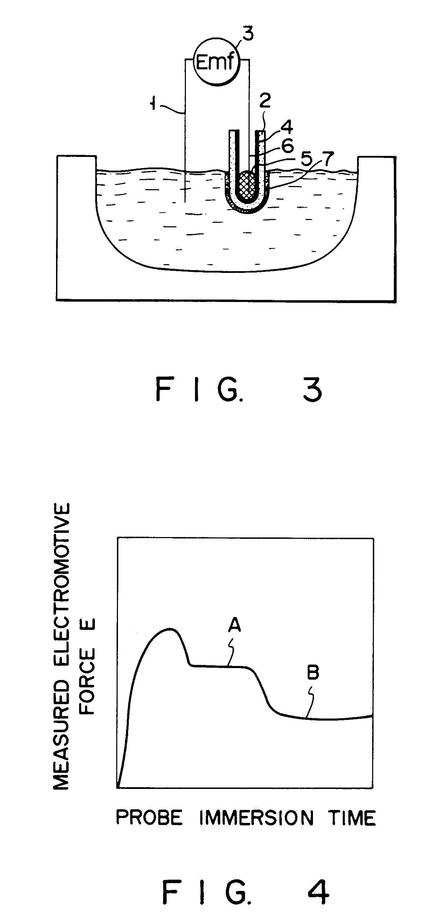

Referring now to Fig. 3, an outline of the sensor will be described.

In Fig. 3, numerals 1, to 7 are denote as follows: 1; lead wire from working electrode 2; coating layer 3; potentio meter 4; electrolyte 5; reference material 6; lead wire from reference electrode 7; local equilibrated zone in molten metal. An assembly constructed of materials is called a sensor bodies. The probe is an electromotive force measuring apparatus which is formed of an electric circuit basically including the lead wire from working electrode 1 and the lead wire from reference electrode 6. Electrolyte 4 may be any solid electrolyte which has oxygen ion conductivity at high temperature and can be used in an ordinary oxygen sensor body. Likewise, reference material 5, which is used to define a reference oxygen partial pressure, may be any substance which can be used in an ordinary oxygen sensor body.

Solid electrolyte 4 has the form of a cylinder, and is charged with reference material 5. The outer surface of solid electrolyte 4 is coated with a coating material which contains an oxide $MO_x$ of the element M to be measured. The activity $a_{MO_x}$ of the oxide $MO_x$ is fixed by the coating material. The electrode 1 and the sensor body 2 having coating layer 7 are immersed in molten metal whose temperature is previously measured by means of a thermocouple or the like.

Preferably, the thickness of the coating layer ranges from 30 to 300 $\mu$m, and most desirably, is about 100 $\mu$m. If the layer thickness is less than 30 $\mu$m, no balance portion can be obtained. If the thickness exceeds 300 $\mu$m, on the other hand, speedy measurement cannot be effected.

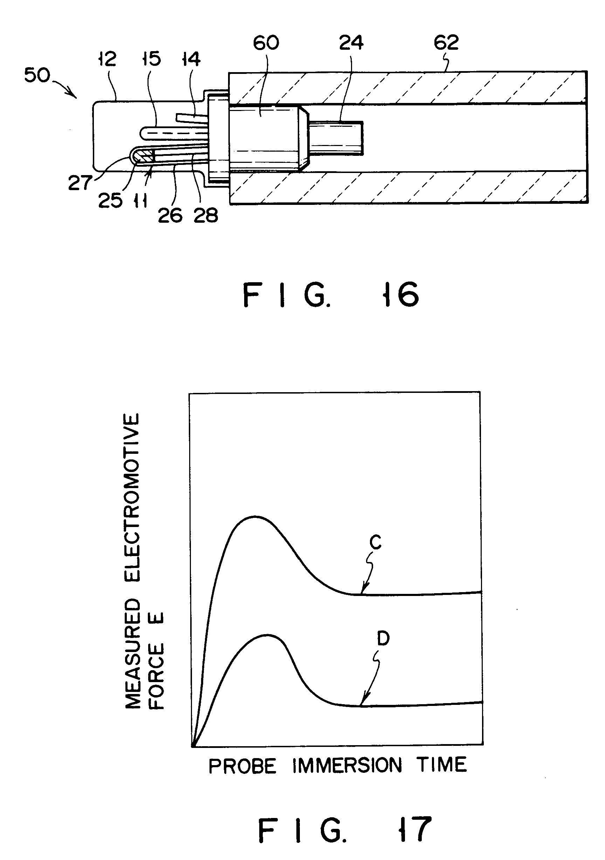

Fig. 17 is a graph showing changes on standing of the electromotive forces E1 and E2 measured by means of different sensor bodies. In Fig. 17, balance portion C represents the electromotive force E2 corresponding to the activity $a_M$ of the solute element M, and balance portion D represents the electromotive force E1 corresponding to the oxygen activity $a_O$.

4

The oxygen activity $a_O$ is obtained according to Equation (1) using the measured electromotive force E1 and the measured temperature T.

$$a_O = K1[(Pe'^{1/4} + P_{O2}^{1/4})\exp(\frac{11.6045E}{T}) - Pe'^{1/4}]^2 \qquad \ldots(1)$$

In Equation (1),

$$K1 = \exp(8.2740 + \frac{16486}{T}) \qquad Pe' = 10^{(24.42 - \frac{74370}{T})}$$

$$P_{O2} = \exp(18.636 - \frac{86384}{T})$$

where $a_O$ is the oxygen activity ($\times 10^{-4}$) of the local balance layer (in the case of the subject element measuring member, first balance portion A or C, which will be described later) or the bulk oxygen activity ($\times 10^{-4}$) (in the case of the oxygen electromotive force measuring member, second balance portion B or C, which will be described later); T, temperature (K) measured by means of the temperature measuring member; K1, conversion factor for oxygen activity and oxygen partial pressure; Pe' electronic conductivity parameter (atm); and $P_{O2}$, oxygen partial pressure (atm) of reference.

Preferably, in this case, a so-called carbon determinator utilizing the solidifying-temperature depression method is used as carbon measuring means. A thermocouple is used for this purpose.

In this case, moreover, the subject element to be measured may be $A\ell$, Si, Mn, Ti, P, Mg, Cr, Ni, or Cu in the molten metal. The activities of these elements are fixed by their respective oxides in coating layer, e.g., $A\ell_2O_3$, $SiO_2$, $MnO$, $TiO_2$, $P_2O_5$, $MgO$, $Cr_2O_3$, $NiO$, or $CuO$.

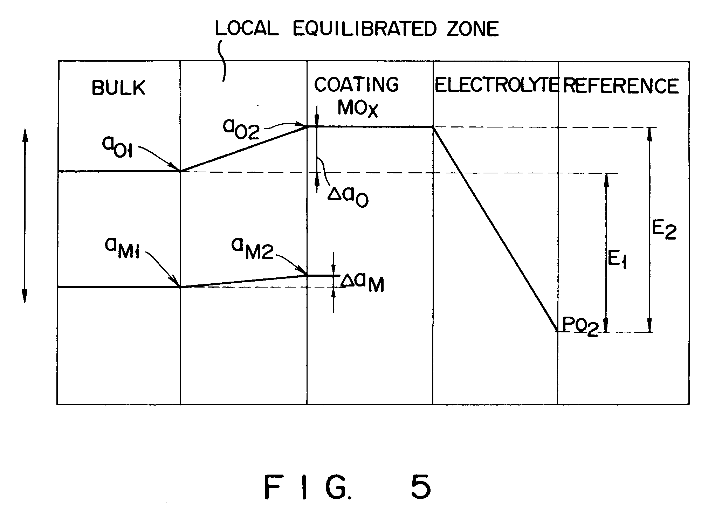

Referring now to Fig. 5, the principle of measurement of the present invention will be described.

When the oxygen sensor, formed of the solid electrolyte coated with $MO_x$, is immersed in the molten metal containing the solute element M, the activity of the element M at the interface between the coating layer and the molten metal is subject to a relation given by

$$M + \frac{x}{2}O_2 = MO_x \qquad \ldots(2)$$

and the local equilibrated zone is formed between the bulk and the coating layer.

In this case, the equilibrium constant K is given by

$$K = \frac{a_{MOx}}{a_{M2} \cdot a_{O2}^{x/2}} \qquad \ldots(3)$$

where $a_{M1}$ and $a_{O1}$ are the activity of the element M and the oxygen activity, respectively, in the molten metal (bulk), and $a_{M2}$ and $a_{O2}$ are the activity of the element M and the oxygen activity, respectively, in the local equilibrated zone.

According to the conventional measuring methods, the activity $a_{M1}$ is regarded as substantially equal to the activity $a_{M2}$. However, if the values of the activity $a_{M1}$ and the oxygen activity $a_{O1}$ resemble each other, or if the oxygen activity $a_{O1}$ varies considerably, the difference between the activities $a_{M1}$ and $a_{M2}$ is great.

Thereupon, substituting Equations (4) and (5) into Equation (3), we obtain Equation (6) as follows:

$$\Delta a_M = a_{M2} - a_{M1} \qquad (4)$$

$$\Delta a_O = a_{O2} - a_{O1} \qquad (5)$$

$$a_{M1} = \frac{a_{MOx}}{K \cdot a_{O2}^{x/2}} - \Delta a_M \qquad \ldots (6)$$

In Equation (6), a Raoultian activity, which is based on the Raoult's law, is used for the activity $a_{MOx}$ of the oxide $MO_x$. Henrian activities, whose standard state is at 1 % by weight, are used individually for the other activities ($a_{M1}$, $a_{M2}$, $a_{O2}$, etc.) than the activity $a_{MOx}$. The Henrian activities are based on the Henry's law.

Since $\Delta a_M$ is produced when the coating layer dissolves, there is a correlation between $\Delta a_M$ and $\Delta a_O$. Accordingly, Equation (6) may be replaced by

$$a_{M1} = \frac{a_{MOx}}{K \cdot a_{O2}^{x/2}} - \alpha \Delta a_O \qquad \ldots (7)$$

In Equation (7), the activity $a_{MOx}$ of the coating layer is regarded as constant, the equilibrium constant K is a function of temperature, and $\Delta a_O$ is the difference between the activities $a_{O1}$ and $a_{O2}$. The mark $\alpha$ is constant. Therefore, the activity $a_{M1}$ can be expressed as follows:

$$a_{M1} = f(a_{O1}, a_{O2}, T) \qquad (8)$$

Hereupon, the activities $a_{O1}$ and $a_{O2}$ are functions of the electromotive forces E1 and E2 detected by means of the sensors and the temperature T. Accordingly, Equation (8) may be replaced by

$$a_{M1} = f(E1, E2, T) \qquad (9)$$

Thus, in the activity measuring method according to the present invention, the electromotive force E1 corresponding to oxygen content in bulk, as well as the electromotive force E2 corresponding to the solute element to be measured and the molten metal temperature T, is measured, and the activity $a_{M1}$ of the subject element is calculated according to Equation (9), using the measured values E1, E2, and T. In this manner, the activity $a_{M1}$ is obtained in consideration of the electromotive force E1, so that higher measuring accuracy can be enjoyed.

If there is a close correlation between the oxygen activity $a_{O1}$ and the dissolved carbon concentration, as in the case of molten iron, for example, activity $a_{O1}$ be expressed as follows:

$$a_{O1} = f([C], T) \qquad (10)$$

Substituting the carbon concentration [C] for the oxygen activity $a_{O1}$ or electromotive force E1 in Equations (8) and (9), therefore, we obtain

$$a_{M1} = f([C], a_{O2}, T) \qquad (11)$$

$$a_{M1} = f([C], E2, T) \qquad (12)$$

Thus, the carbon concentration [C] is detected in place of the electromotive force E1, and the activity $a_{M1}$ of the solute element to be measured is calculated according to Equation (12), using the carbon concentration [C], the electromotive force E2, and the molten metal temperature T.

According to another aspect of the present invention, there is provided a probe for measuring the activity of a solute element in molten metal, which comprises: a sensor body for solute element measurement; and a lead wire from working electrode connected electrically to the sensor body with coating layer and constituting an electromotive force measuring circuit. The sensor body for solute element measurement is constructed of cylindrical solid electrolyte having oxygen ion conductivity, and packing a reference material, and peripherally covered by a coating layer composed of an oxide containing a solute element M to be measured or a composite oxide thereof. The coating layer is composed of the oxide or the composite oxide having a composition such that two or more balance portions are provided for an electromotive force measured by the electromotive force measuring circuit.

Fig. 4 is a graph showing the change on standing of the electromotive force E measured by means of the probe. In Fig. 4, the axis of abscissa represents the time elapsed after the start of measurement, and the axis of ordinate represents the measured electromotive force E. When the probe is immersed in the molten metal, the individual members are heated so that the electromotive force E attains its peak. Thereafter, the members are thermally stabilized, and first balance portion A appears on the electromotive force curve. First balance portion A continues so long as the coating layer remains on the sensor body. As the coating layer gradually melts into the molten metal, it ceases to be able to regulate the oxygen activity of a local equilibrated zone, and the measured electromotive force E gradually approaches the bulk oxygen activity. Thus, the measured electromotive force E shifts from first balance portion A to second balance portion B, whereupon it stabilizes. In this case, the electromotive force E at first balance portion A corresponds to the electromotive force E1 defined by the solute element content in the molten metal, while electromotive force E at second balance portion B corresponds to electromotive force E2 defined by oxygen content in the bulk of molten metal. Thus, balance portions A and B of Fig. 4 correspond to balance portions C and B of Fig. 17. Preferably, in this case, the coating layer is adjusted to a suitable thickness. If the coating layer is too thin, for example, it will disappear before the first balance portion is created. If it is too thick, on the other hand, it will be a long time before the second balance portion is created.

If two or more coating layers are used to cover the sensor body, the number of balance portions for the measured electromotive force E can be increased, so that the electromotive forces corresponding to several solute elements contents can be measured.

This invention can be more fully understood from the following detailed description when taken in conjunction with the accompanying drawings, in which:

Figs. 1 and 2 are graphs prepared individually by plotting measurement results obtained using prior art activity measuring methods;

Fig. 3 is a schematic view for illustrating the principle of electromotive force measurement;

Fig. 4 is a graph showing a change on standing of the measured electromotive force;

Fig. 5 is a diagram for illustrating the principle of measurement of the activity of a solute element in molten metal;

Fig. 6 is a block diagram showing an outline of an activity measuring system;

Fig. 7 is a longitudinal sectional view schematically showing a probe used in an activity measuring method according to a first embodiment of the present invention;

Fig. 8 is a longitudinal sectional view showing a sensor body for measuring the solute element activity;

Figs. 9 to 11 are graphs prepared individually by plotting measurement results according to the first embodiment;

Fig. 12 is a longitudinal sectional view schematically showing a probe used in an activity measuring method according to a second embodiment of the invention;

Figs. 13 to 15 are graphs prepared individually by plotting measurement results according to the second embodiment;

Fig. 16 is a longitudinal sectional view schematically showing an activity measuring probe (probe having two balance portions) according to another embodiment of the invention; and

Fig. 17 is a graph showing changes on standing of the electromotive forces E1 and E2 measured by means of different sensor bodies.

Various embodiments of the present invention will now be described in detail with reference to the accompanying drawings.

The following is a description of measurement of the activity of Mn in molten metal.

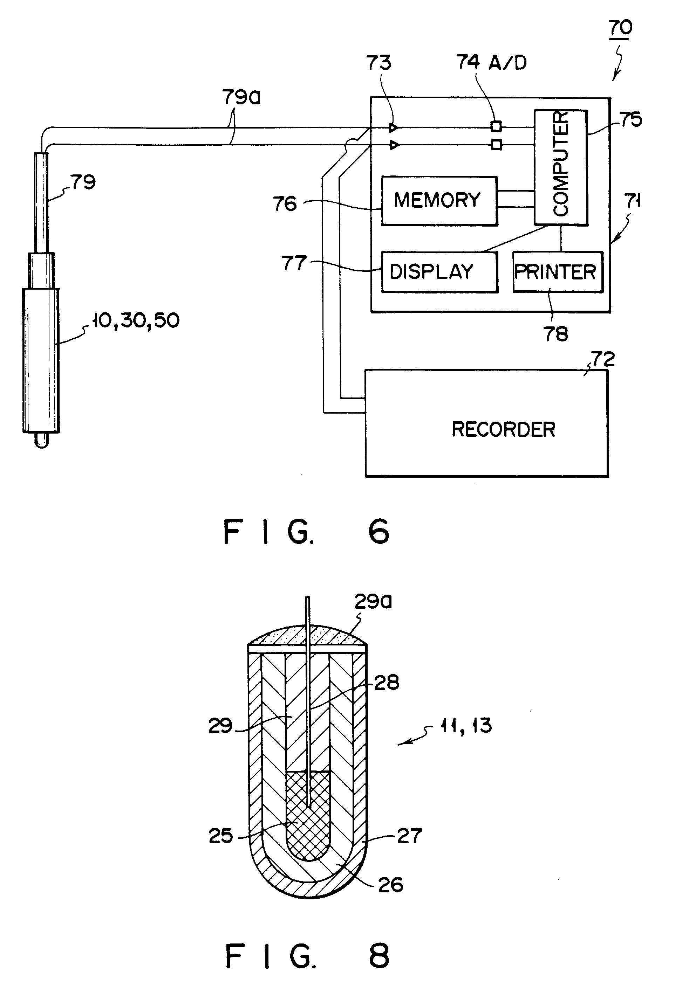

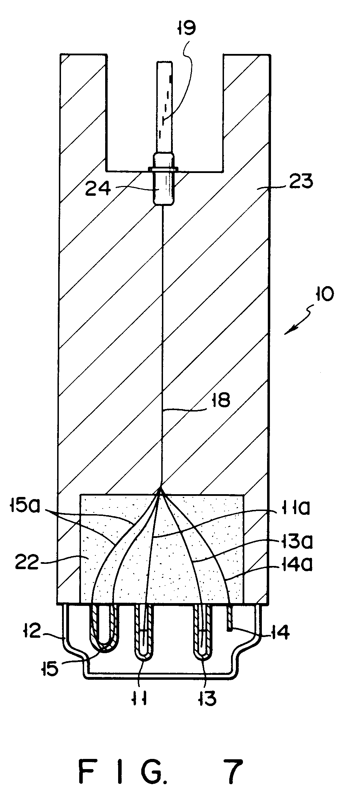

As shown in Fig. 6, activity measuring system 70 is formed of arithmetic unit 71, recorder 72, and probe 10 (30, 50) connected to one another. Probe 10 (30, 50) is attached to the distal end of holder 79. Holder 79, which constitutes part of a sub-lance system of a converter, is supported over the converter by means of a lift system (not shown). When holder 79 is lowered by the lift system, probe 10 is immersed in the molten metal in the converter. The proximal portion of holder 79 is connected to the respective input sections of arithmetic unit 71 and recorder 72 by means of cables 79a. Unit 71 comprises computer 75 and peripheral apparatuses, including memory 76, display 77, and printer 78. An automatic balance system is used as the recording system for recorder 72. In arithmetic unit 71, an electromotive force detected by means of probe 10 is supplied, as a voltage signal, to the input section of computer 75 through amplifier 73 and A/D converter 74.

Referring now to Fig. 7, probe 10 according to a first embodiment will be described. Probe 10 is used singly to measure three quantities of state; the electromotive force according to manganese content E2, the electromotive force according to oxygen content E1, and the molten metal temperature T. Thus, in probe 10, the electromotive forces E1 and E2 are measured by means of different sensor bodies.

Probe 10 includes cylindrical protecting tube 23, various sensors (bodies, wires etc.) 11, 13, 14 and 15 projecting from the distal end of tube 23, and connector 24 provided at the proximal end portion of tube 23 so as to be connected electrically to holder 79. The respective proximal portions of sensors (bodies, wires etc.) 11, 13, 14 and 15 are embedded in refractory cement 22. Lead wire 18 for sensors 11, 13, 14 and 15 is connected to connector 24. A hollow is formed in the connector-side portion of protecting tube 23, and contact member 19 of connector 24 projects into the hollow. Thus, when probe 10 is attached to holder 79, member 19 is connected to the cables of holder 79, so that the voltage signal corresponding to the detected electromotive force can be stored in memory 76 through computer 75.

Various sensor bodies of the probe will now be described in detail.

Sensor body 11 is used to measure the manganese electromotive force. As shown in Fig. 8, sensor body 11, which is in the form of a tube closed at one end, is composed of reference material 25 formed of a material having a known oxygen partial pressure, solid electrolyte 26 having oxygen ion conductivity and holding the reference material inside, and coating layer 27 covering the electrolyte. One end of lead wire 28 is immersed in reference material 25. A space over reference material 25 is stuffed with closed tube 29, formed of a quartz or aluminum tube, and is further closed by means of cement 29a.

In this case, a powder mixture of chromium (Cr) and chromium oxide ($Cr_2O_3$), for example, is used for reference material 25. Partially stabilized zirconia, such as $ZrO_2$-MgO (8 mol %), is used for solid electrolyte 26. Preferably, electrolyte 26 has an inner diameter of 3.0 mm, outer diameter of 4.7 mm, and length of 35 mm. Preferably, moreover, a mixture of manganese oxide (MnO) and an organic binder is used for coating layer 27.

Sensor body 13, which is used to measure the electromotive force corresponding to oxygen content is constructed in the same manner as sensor body 11, except the absence of coating layer 27.

Sensor body 11 and lead wire from working electrode 14, along with their corresponding lead wires 11a and 14a, constitute one potential difference circuit, while sensor body 13 and lead wire from working electrode 14, along with their corresponding lead wires 13a and 14a, constitute another potential difference circuit.

Sensor 15 is a thermocouple formed of platinum-rhodium (Pt-Rh) wire.

The following is a description of the results of measurement of the activity of manganese in converter molten steel, by the use of the aforementioned activity measuring system.

Measuring conditions include the molten steel temperature of 1,580 to 1,720°C, manganese activity of 0 to 2, oxygen activity of 30 to 500 ($\times 10^{-4}$), carbon concentration of 0.04 to 0.50 % by weight, and measuring time of about 10 seconds.

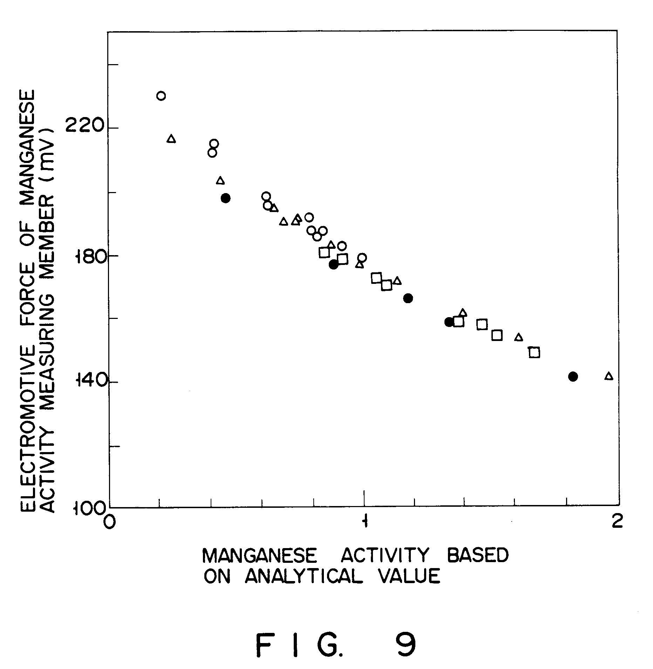

Fig. 9 is a graph showing the relationship between the manganese activity (axis of abscissa) based on analytical values and the electromotive force E2 (axis of ordinate) of a manganese sensor. In Fig. 9, circles, triangles, squares, and black dots correspond to the ranges of 2.0 to 2.7, 2.7 to 3.3, 3.3 to 4.0, and 4.0 to 5.3, respectively, in terms of the ratio of the oxygen activity $a_{O2}$ measured by means of a manganese sensor to the oxygen activity $a_{O1}$ measured by means of an oxygen sensor. As seen from this graph, the electromotive force is influenced by the ratio between the bulk oxygen activity ($a_{O1}$) and the oxygen value ($a_{O2}$) of the manganese sensor.

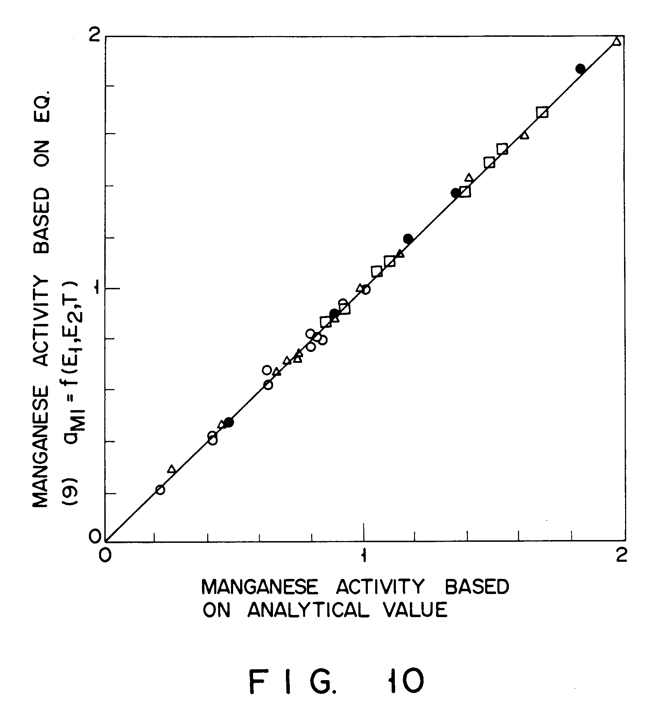

Fig. 10 is a graph showing the relationship between the manganese activity (axis of abscissa) based on analytical values and the manganese activity (axis of ordinate) obtained according to Equation (9). The symbols in Fig. 10 are used in the same manner as those of Fig. 9. As seen from Fig. 10, the measured values obtained with use of probe 10 fully agree with the analytical values, without any substantial exceptions.

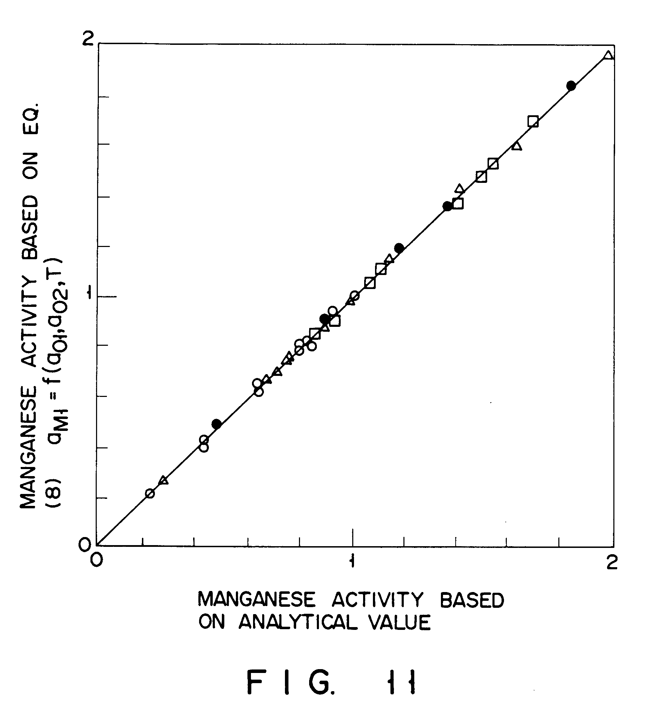

Fig. 11 is a graph showing the relationship between the manganese activity (axis of abscissa) based on analytical values and the manganese activity (axis of ordinate) obtained according to Equation (8). The symbols in Fig. 11 are used in the same manner as those of Fig. 9. As seen from Fig. 11, the measured values obtained with use of probe 10 fully agree with the analytical values, without any substantial exceptions. According to a conventional method, in this connection, the relationships between the measured values and the analytical values are subject to substantial dispersion, as shown in Figs. 1 and 2.

According to the first embodiment described above, correlation coefficient r (no dispersion in the case of r = 1.00) was able to be increased from 0.990 for the conventional case to the level of 0.997 to 0.998. As for estimated accuracy (standard deviation) $\sigma$, it was able to be improved from ±0.085 for the conventional case to the level of ±0.027 to ±0.029.

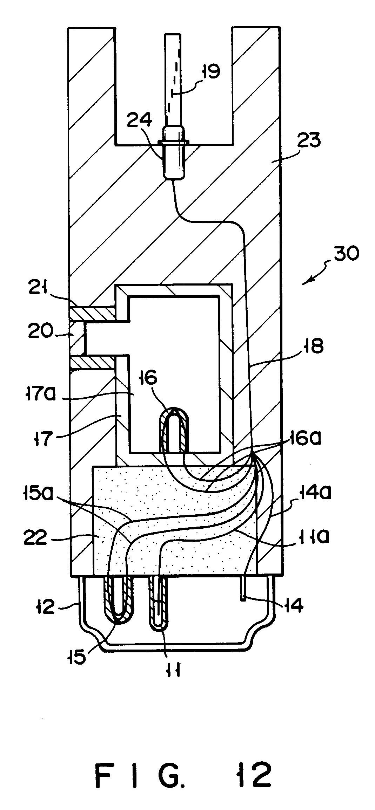

Referring now to Fig. 12, probe 30 according to a second embodiment of the present invention will be described. Probe 30 is used to measure three quantities of state; the electromotive force E2 according to manganese content, the carbon concentration [C] of molten steel, and the molten steel temperature T. A description of the portions common to the first and second embodiments is omitted.

Probe 30 of the second embodiment has sample chamber 17a in protecting tube 23. The inlet of chamber 17a is closed by means of cap 20. Carbon sensor 16 is provided inside chamber 17a. Cap 20 is formed of paper or other material which can be destroyed by the heat of the molten steel. Carbon sensor 16 is a thermocouple which resembles temperature sensor 15. When cap 20 is melted to allow the molten steel to penetrate into sample chamber 17a and solidify therein, the solidifying temperature is measured by sensor 16. The carbon concentration [C] is obtained on the basis of the solidifying temperature. Sensors (bodies, wires etc.) 11, 14, 15 and 16 are connected to main lead wire 18 by means of lead wires 11a, 14a, 15a and 16a, respectively.

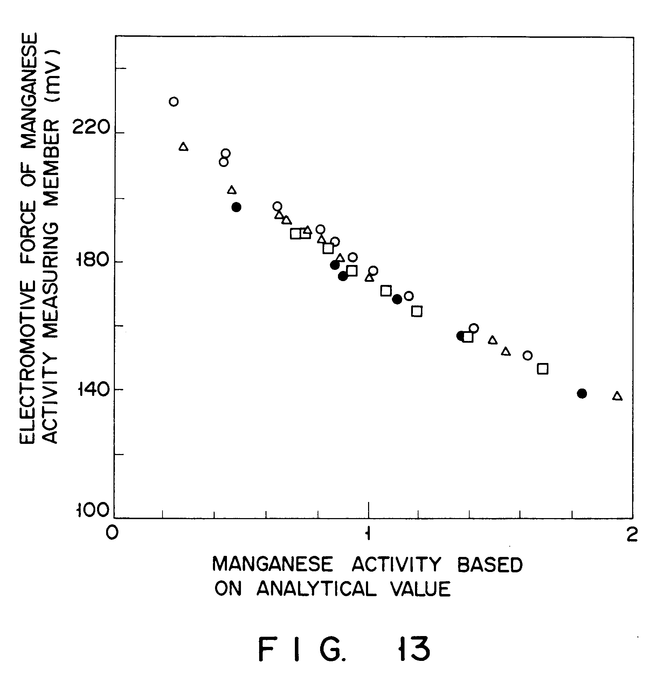

Fig. 13 is a graph showing the relationship between the manganese activity (axis of abscissa) based on analytical values and the electromotive force E2 (axis of ordinate) of the manganese sensor. In Fig. 13, circles, triangles, squares, and black dots correspond to the ranges of 0.04 to 0.1, 0.1 to 0.2, 0.2 to 0.3, and 0.3 to 0.5, respectively, in terms of the carbon concentration (% by weight) measured by means of a carbon sensor. As seen from this graph, the electromotive force is influenced by the carbon concentration.

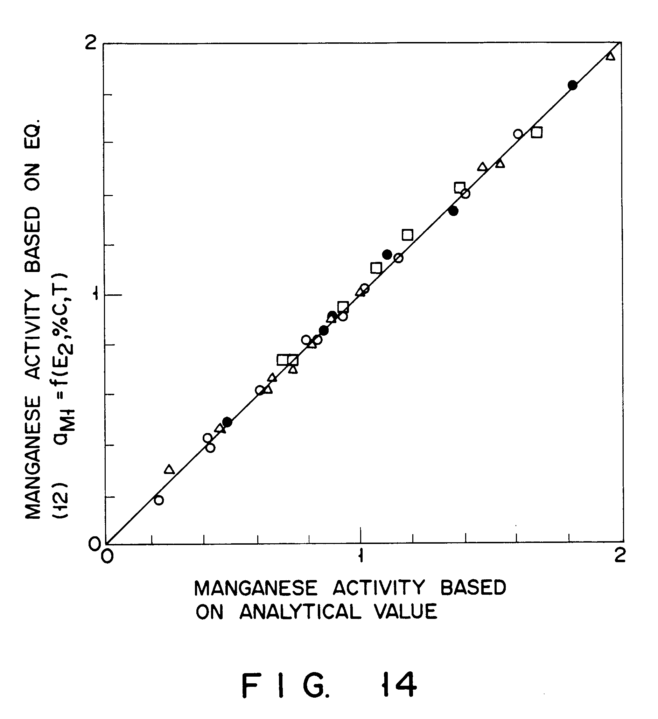

Fig. 14 is a graph showing the relationship between the manganese activity (axis of abscissa) based on analytical values and the manganese activity (axis of ordinate) obtained according to Equation (12). The symbols in Fig. 14 are used in the same manner as those of Fig. 13. As seen from Fig. 14, the measured values obtained with use of probe 30 fully agree with the analytical values, without any substantial exceptions.

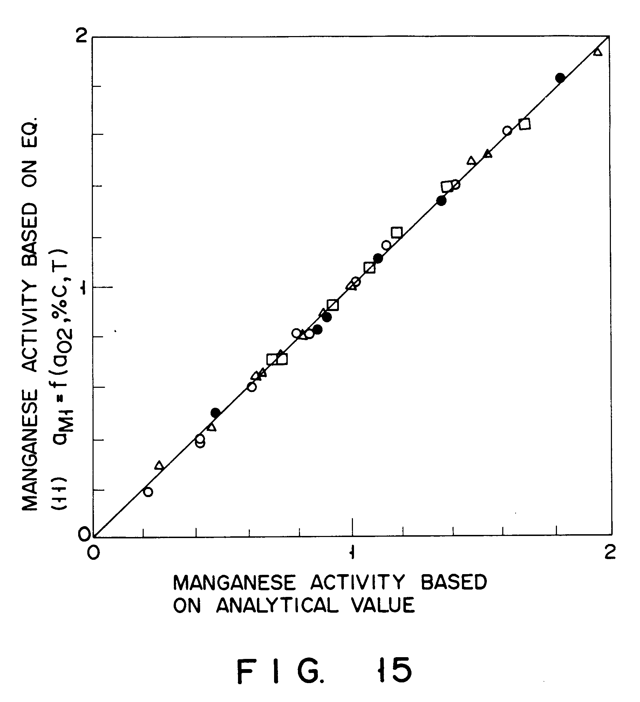

Fig. 15 is a graph showing the relationship between the manganese activity (axis of abscissa) based on analytical values and the manganese activity (axis of ordinate) obtained according to Equation (11). The symbols in Fig. 15 are used in the same manner as those of Fig. 13. As seen from Fig. 15, the measured values obtained with use of probe 30 fully agree with the analytical values, without any substantial exceptions.

According to the second embodiment described above, correlation coefficient $r$ (no dispersion in the case of r = 1.00) was able to be increased from 0.988 for the conventional case to 0.997. As for estimated accuracy (standard deviation) $\sigma$, it was able to be improved from ±0.091 for the conventional case to the level of ±0.029 to ±0.030.

Referring now to Fig. 16, there will be described probe 50 according to another embodiment of the present invention, having two balance portions, and a method for measuring the activity of Mn in molten steel by means of probe 50.

Probe 50 has a lead wire from working electrode 14, sensor body 11, thermocouple 15 at its distal end. Electrode 14 sensor body 11 and thermocouple 15, which are covered by means of cap 12, are connected to connector 24 by means of housing 60. Housing 60, which is fitted in protecting tube 62, serves to fix lead wires of body 11 and from working electrode 14 and thermocouple 15 to tube 62. When probe 50 is attached to the distal end of a sub-lance (not shown), connector 24 is connected to a connector (not shown) of a holder. Thereupon, lead wires of body 11 and from working electrode 14, and thermocouple 15 are connected to a potentiometer (not shown).

Lead wire from working electrode 14 is formed of a molybdenum rod of 3-mm in diameter. Sensor body 11 includes solid electrolyte 26, reference material 25, and lead wire 28 from reference electrode. Electrolyte 26, which is in the form of a tube closed at one end, is charged with reference material 25. The electrolyte is composed of $ZrO_2$ containing 8 mol % of MgO. Reference material 25 is a powder mixture of Cr and $Cr_2O_3$. The mixture ratio of Cr to $Cr_2O_3$ is about 98 to 2. Lead wire 28 from reference material is formed of a molybdenum wire of 0.3-mm in diameter.

The outer surface of solid electrolyte 26 is covered with coating layer 27. Layer 27 is formed by applying a mixture of an oxide $MO_x$ (e.g., MnO) and a binder and the like to the outer surface of electrolyte 26. The average thickness of layer 27 is 100 $\mu$m. Coating layer 27 may be porous or nonporous, provided it can uniformly melt all over.

When probe 50 is immersed in the molten steel, coating layer 27 melts into the steel. While layer 27 is melting, the electrode potential is kept constant, and the electromotive force produced between lead wires of body 11 and from working electrode 14 undergoes no change. In this case, first balance portion A of electromotive force, which corresponds to the manganese content, appears, as shown in Fig. 4.

Some time after the start of the measurement, coating layer 27 disappears, so that solid electrolyte 26 is exposed. As a result, a new electromotive force is produced between lead wires of body 11 and from working electrode 14, and second balance portion B of electromotive force, which corresponds to the oxygen content in bulk, appears, as shown in Fig. 4.

According to probe 50 described above, the manganese activity $a_{Mn}$ and the oxygen activity $a_O$ can be obtained by detecting both the manganese and oxygen electromotive forces by means of only paired lead wires of body 11 and from working electrode 14. Thus, even if a plurality of solute elements must be subjected to measurement, the sensor bodies attached to the distal end of the probe are not increased in number, so that the diameter of the probe can be reduced. The outer diameter of probe 50 is 35 mm, which is shorter than 50 mm for the conventional probe.

The following is a general description of the effect of the present invention.

According to the activity measuring method of the present invention, the activities of elements to be measured, such as Mn, Si, P, Cr, etc., can be seized more quickly with higher accuracy by correcting them by means of the bulk oxygen activity. According to the conventional measuring method, the estimated accuracy of the activity is too low for some conditions unless the activity is corrected by means of the oxygen activity $a_{O1}$. Even in these conditions, that is, if the bulk oxygen activity $a_{O1}$ is approximate to the activity $a_{M1}$ of the solute element or varies considerably, for example, the method of the invention ensures measurement.

According to the probe of the present invention, moreover, the electromotive forces of a plurality of solute elements can be measured without increasing the number of sensor bodies, so that the diameter of the probe can be reduced. Thus, the thermal capacity of the whole probe is reduced, so that the sensor bodies can attain thermal equilibrium in a short period of time, and there is no thermodynamic interference between the adjacent bodies. Accordingly, the electromotive force according to the solute element content can be detected quickly and securely, and the activity can be obtained on the basis of the detected value.

If two or more coating layers are used to cover the sensor body, furthermore, the electromotive forces of two or more solute elements can be detected by means of only one sensor bodies and a lead wire from working electrode.

Probes for measuring the manganese activity $a_{Mn}$ and the phosphorus activity $a_p$ may be used in a sublance system for converter refining.

Further, a probe for measuring the silicon activity $a_{Si}$ may be used in a ladle furnace process.

## Claims

1. A method for measuring an activity of a solute element M in molten metal, comprising steps of:

    measuring an electromotive force E1 corresponding to oxygen content of the molten metal by means of a solid electrolyte (26) having oxygen ion conductivity and measuring temperature T of the molten metal;

    measuring an electromotive force E2 corresponding to the solute element content to be measured in the molten metal by means of a solid electrolyte (26) having oxygen ion conductivity and coated with an oxide containing the solute element M to be measured; and

    obtaining the activity $a_M$ of the solute element M to be measured on the basis of the measured electromotive forces E1, E2 and the measured temperature T.

2. The activity measuring method according to claim 1, characterized in that said solute element M to be measured is manganese.

3. The activity measuring method according to claim 1, characterized in that said solute element M to be measured is silicon.

4. The activity measuring method according to claim 1, characterized in that said solute element M to be measured is phosphorus.

5. The activity measuring method according to claim 1, characterized in that said solute element M to be measured is chromium.

6. A method for measuring an activity of a solute element M in molten metal, comprising steps of:

    measuring a temperature T and a solidifying temperature Ts of the molten metal;

    obtaining the carbon concentration [C] of the molten metal on the basis of the solidifying temperature Ts;

    measuring the electromotive force E2 corresponding to the solute element content to be measured in the molten metal by means of a solid electrolyte (26) having oxygen ion conductivity and coated with an oxide containing the solute element M to be measured; and

    obtaining the activity $a_M$ of the solute element M to be measured on the basis of the measured electromotive force E2, the carbon concentration [C], and the measured temperature T.

7. The activity measuring method according to claim 6, characterized in that said solute element M to be measured is manganese.

8. The activity measuring method according to claim 6, characterized in that said solute element M to be measured is silicon.

9. The activity measuring method according to claim 6, characterized in that said solute element M to be measured is phosphorus.

10. The activity measuring method according to claim 6, characterized in that said solute element M to be measured is chromium.

11. A probe for measuring an activity of a solute element M in molten metal, comprising:
   a sensor body (11, 13) for solute element measurement; and
   a lead wire from working electrode (14) connected electrically to the sensor body (11, 13) and constituting an electromotive force measuring circuit,
   said sensor (11, 13) body for solute element measurement including a solid electrolyte surrounding a reference (25) and having oxygen ion conductivity, and a coating layer (27) composed of an oxide containing a solute element M to be measured or a composite oxide thereof and covering the outer periphery of the solid electrolyte (26),
   said oxide or said composite oxide for said coating layer (27) having a composition such that two or more balance portions are provided for an electromotive force E1, E2 measured by the electromotive force measuring circuit,
   the electromotive force E1, E2 being defined by the activity $a_M$ of the solute element M to be measured in the first one of the balance portions, and by the oxygen activity $a_O$ in the molten metal in the last balance portion.

12. The activity measuring probe according to claim 11, further comprising a thermocouple (15, 16) for measuring the temperature T of the molten metal.

13. The activity measuring probe according to claim 11, characterized in that said coating layer (27) of said sensor body (11, 13) for solute element measurement contains manganese oxide or a composite oxide thereof.

14. The activity measuring probe according to claim 13, characterized in that the thickness of said coating layer (27) ranges from 30 to 300 $\mu$m.

15. The activity measuring probe according to claim 11, characterized in that said coating layer (27) of said sensor body (11, 13) for solute element measurement contains silicon oxide or a composite oxide thereof.

16. The activity measuring probe according to claim 15, characterized in that the thickness of said coating layer (27) ranges from 30 to 300 $\mu$m.

17. The activity measuring probe according to claim 11, characterized in that said coating layer (27) of said sensor body (11, 13) for solute element measurement contains phosphorus oxide or a composite oxide thereof.

18. The activity measuring probe according to claim 17 characterized in that the thickness of said coating layer (27) ranges from 30 to 300 $\mu$m.

19. The activity measuring probe according to claim 11, characterized in that said coating layer (27) of said sensor body (11, 13) for solute element measurement contains chromium oxide or a composite oxide thereof.

20. The activity measuring probe according to claim 19, characterized in that the thickness of said coating layer ranges from 30 to 300 $\mu$m.

**21.** An apparatus for measuring an activity of a solute element M in molten metal, comprising:

temperature measuring means (15, 16) for measuring the temperature of the molten metal; and

probe means (10, 30, 50) for measuring an electromotive forces E1 corresponding to oxygen content and E2 corresponding to solute element content of the molten metal,

said probe means (10, 30, 50) including a sensor body (11, 13) for solute element measurement, and a lead wire from working electrode (14) connected electrically to the sensor body (11, 13) and constituting an electromotive force measuring circuit, said sensor body (11, 13) for solute element measurement including a solid electrolyte (26) surrounding a reference material (25) and having oxygen ion conductivity, and a coating layer (27) composed of an oxide containing a solute element M to be measured or a composite oxide thereof and covering the outer periphery of the solid electrolyte (26), said oxide or said composite oxide for said coating layer having a composition such that two or more balance portions are provided for an electromotive force E1, E2 measured by the electromotive force measuring circuit, the electromotive force E1, E2 being defined by the activity $a_M$ of the solute element M to be measured in the first one of the balance portions, and by the oxygen activity $a_O$ in the molten metal in the last balance portion.

**Patentansprüche**

**1.** Verfahren zur Messung der Aktivität eines gelösten Elementes bzw. Beimengungselementes M in einem geschmolzenen Metall, welches folgenden Schritte aufweist:

- Messen der elektromotorischen Kraft E1, die dem Sauerstoffgehalt des geschmolzenen Metalls entspricht, mittels eines festen Elektrolyten (26), der eine Sauerstoffionenleitfähigkeit besitzt, und Messen der Temperatur T des geschmolzenen Metalls;
- Messen elektromotorischen Kraft E2, die dem Gehalt des zu messenden Beimengungselementes in dem geschmolzenen Metall entspricht, mittels eines festen Elektrolyten (26), der eine Sauerstoffionenleitfähigkeit besitzt und mit einem Oxid beschichtet ist, welches das zu messende Beimengungselement M enthält; und
- Erhalten der Aktivität $a_M$ des zu messenden Beimengungselementes M auf der Basis der gemessenen elektromotorischen Kräfte E1, E2 und der gemessenen Temperatur T.

**2.** Verfahren zur Messung der Aktivität gemäß Anspruch 1, dadurch gekennzeichnet, daß das zu messende Beimengungselement M Mangan ist.

**3.** Verfahren zur Messung der Aktivität gemäß Anspruch 1, dadurch gekennzeichnet, daß das zu messende Beimengungselement M Silicium ist.

**4.** Verfahren zur Messung der Aktivität gemäß Anspruch 1, dadurch gekennzeichnet, daß das zu messende Beimengungselement M Phosphor ist.

**5.** Verfahren zur Messung der Aktivität gemäß Anspruch 1, dadurch gekennzeichnet, daß das zu messende Beimengungselement M Chrom ist.

**6.** Verfahren zur Messung der Aktivität eines gelösten Elementes bzw. Beimengungselementes M in einem geschmolzenen Metall, welches folgende Schritte aufweist:

- Messen einer Temperatur T und einer Verfestigungstemperatur Ts des geschmolzenen Metalls;
- Erhalten der Kohlenstoffkonzentration [C] des geschmolzenen Metalls auf der Basis der Verfestigungstemperatur Ts;
- Messen der elektromotorischen Kraft E2, die dem Gehalt des zu messenden Beimengungselementes in dem geschmolzenen Metall entspricht, mittels eines festen Elektrolyten (26), der eine Sauerstoffionenleitfähigkeit besitzt und mit einem Oxid beschichtet ist, welches das zu messende Beimengungselement M enthält; und
- Erhalten der Aktivität $a_M$ des zu messenden Beimengungselementes M auf der Basis der gemessenen elektromotorischen Kraft E2, der Kohlenstoffkonzentration [C] und der gemessenen Temperatur T.

EP 0 358 168 B1

7. Verfahren zur Messung der Aktivität gemäß Anspruch 6,
dadurch gekennzeichnet,
daß das zu messende Beimengungselement M Mangan ist.

8. Verfahren zur Messung der Aktivität gemäß Anspruch 6,
dadurch gekennzeichnet,
daß das zu messende Beimengungselement M Silicium ist.

9. Verfahren zur Messung der Aktivität gemäß Anspruch 6,
dadurch gekennzeichnet,
daß das zu messende Beimengungselement M Phosphor ist.

10. Verfahren zur Messung der Aktivität gemäß Anspruch 6,
dadurch gekennzeichnet,
daß das zu messende Beimengungselement M Chrom ist.

11. Sonde zur Messung der Aktivität eines gelösten Elementes bzw. Beimengungselementes M in einem geschmolzenen Metall, umfassend:
- einen Sensorkörper (11, 13) für die Messung des Beimengungselementes; und
- einen Leitungsdraht von der Arbeitselektrode (14), der elektrisch mit dem Sensorkörper (11, 13) verbunden ist und der eine Meßschaltung für die elektromotorische Kraft bildet;
wobei der Sensorkörper (11, 13) für die Messung des Beimengungselementes einen festen Elektrolyten, der ein Referenzmaterial (25) umgibt und eine Sauerstoffionenleitfähigkeit besitzt, und eine Überzugsschicht (27) aufweist, die aus einem Oxid, das ein zu messendes Beimengungselement M enthält, oder aus einem gemischten Oxid davon besteht, und die den Außenumfang des festen Elektrolyten (26) umgibt;
wobei das Oxid oder das gemischte Oxid für die Überzugsschicht (27) eine Zusammensetzung besitzt, so daß zwei oder mehr Balance-Bereiche für eine elektromotorische Kraft E1, E2 vorgesehen sind, welche mittels der Meßschaltung für die elektromotorische Kraft gemessen wird; und wobei die elektromotorische Kraft E1, E2 durch die Aktivität $a_M$ des zu messenden Beimengungselementes M in dem ersten der Balance-Bereiche und durch die Sauerstoffaktivität $a_O$ in dem geschmolzenen Metall in dem letzten Balance-Bereich definiert wird.

12. Sonde zur Aktivitätsmessung gemäß Anspruch 11,
welche ferner ein Thermoelement (15, 16) zur Messung der Temperatur T des geschmolzenen Metalls enthält.

13. Sonde zur Aktivitätsmessung gemäß Anspruch 11,
dadurch gekennzeichnet,
daß die Überzugsschicht (27) des Sensorkörpers (11, 13) für die Messung des Beimengungselementes Manganoxid oder ein gemischtes Oxid davon enthält.

14. Sonde zur Aktivitätsmessung gemäß Anspruch 13,
dadurch gekennzeichnet,
daß die Dicke der Überzugsschicht (27) zwischen 30 und 300 $\mu$m liegt.

15. Sonde zur Aktivitätsmessung gemäß Anspruch 11,
dadurch gekennzeichnet,
daß die Überzugsschicht (27) des Sensorkörpers (11, 13) für die Messung des Beimengungselementes Siliciumoxid oder ein gemischtes Oxid davon enthält.

16. Sonde zur Aktivitätsmessung gemäß Anspruch 15,
dadurch gekennzeichnet,
daß die Dicke der Überzugsschicht (27) zwischen 30 und 300 $\mu$m liegt.

17. Sonde zur Aktivitätsmessung gemäß Anspruch 11,
dadurch gekennzeichnet,
daß die Überzugsschicht (27) des Sensorkörpers (11, 13) für die Messung des Beimengungselementes

13

Phosphoroxid oder ein gemischtes Oxid davon enthält.

**18.** Sonde zur Aktivitätsmessung gemäß Anspruch 17,
dadurch gekennzeichnet,
daß die Dicke der Überzugsschicht (27) zwischen 30 und 300 $\mu$m liegt.

**19.** Sonde zur Aktivitätsmessung gemäß Anspruch 11,
dadurch gekennzeichnet,
daß die Überzugsschicht (27) des Sensorkörpers (11, 13) für die Messung des Beimengungselementes
Chromoxid oder ein gemischtes Oxid davon enthält.

**20.** Sonde zur Aktivitätsmessung gemäß Anspruch 19,
dadurch gekennzeichnet,
daß die Dicke der Überzugsschicht zwischen 30 und 300 $\mu$m liegt.

**21.** Vorrichtung zur Messung der Aktivität eines gelösten Elementes bzw. Beimengungselementes M in
einem geschmolzenen Metall, die folgendes aufweist:
- eine Temperaturmeßeinrichtung (15, 16) zur Messung der Temperatur des geschmolzenen
Metalls; und
- eine Sondeneinrichtung (10, 30, 50) zur Messung einer elektromotorischen Kraft E1, die dem
Sauerstoffgehalt entspricht, und einer elektromotorischen Kraft E2, die dem Gehalt an Beimengungselement des geschmolzenen Metalls entspricht,
wobei die Sondeneinrichtung (10, 30, 50) einen Sensorkörper (11, 13) für die Messung eines
Beimengungselementes und einen Leitungsdraht von der Arbeitselektrode (14) aufweist, der
elektrisch mit dem Sensorkörper (11, 13) verbunden ist und eine Meßschaltung für die elektromotorische Kraft bildet, wobei der Sensorkörper (11, 13) für die Messung des Beimengungselementes einen festen Elektrolyten (26), der ein Referenzmaterial (25) umgibt und eine Sauerstoffionenleitfähigkeit besitzt, und eine Überzugsschicht (27) aufweist, die aus einem Oxid, das ein zu
messendes Beimengungselement M enthält, oder einem gemischten Oxid davon besteht, und die
den Außenumfang des festen Elektrolyten (26) umgibt, wobei das Oxid oder das gemischte Oxid
für die Überzugsschicht (27) eine Zusammensetzung besitzt, so daß zwei oder mehr Balance-
Bereiche für eine elektromotorische Kraft E1, E2 vorgesehen sind, welche mittels der Meßschaltung für die elektromotorische Kraft gemessen wird, wobei die elektromotorische Kraft E1, E2
durch die Aktivität $a_M$ des zu messenden Beimengungselementes M in dem ersten der Balance-
Bereiche und durch die Sauerstoffaktivität $a_O$ in dem geschmolzenen Metall in dem letzten
Balance-Bereich definiert wird.

**Revendications**

**1.** Procédé pour mesurer l'activité d'un élément soluté (M) dans du métal fondu, comprenant les étapes
consistant à :
- mesurer une force électromotrice E1 qui correspond à la teneur en oxygène du métal fondu, au
moyen d'un électrolyte solide (26) qui présente une conductivité vis-à-vis des ions oxygène, et à
mesurer la température (T) du métal fondu ;
- mesurer une force électromotrice E2 qui correspond à la teneur en élément soluté à mesurer
dans le métal fondu au moyen d'un électrolyte solide (26) présentant une conductivité vis-à-vis
des ions oxygène et qui est revêtu d'un oxyde contenant l'élément soluté M à mesurer; et
- obtenir l'activité $a_M$ de l'élément soluté M à mesurer en se basant sur les forces électromotrices
mesurées E1, E2 et sur la température mesurée T.

**2.** Procédé de mesure selon la revendication 1, caractérisé en ce que ledit élément soluté M à mesurer
est du manganèse.

**3.** Procédé de mesure selon la revendication 1, caractérisé en ce que ledit élément soluté M à mesurer
est du silicium.

**4.** Procédé de mesure selon la revendication 1, caractérisé en ce que ledit élément soluté M à mesurer
est du phosphore.

14

5. Procédé de mesure selon la revendication 1, caractérisé en ce que ledit élément soluté M à mesurer est du chrome.

6. Procédé pour mesurer l'activité d'un élément soluté M dans du métal fondu, comprenant les étapes consistant à:
   - mesurer une température T et une température de solidification Ts du métal fondu ;
   - obtenir la concentration en carbone [C] du métal fondu sur la base de la température de solidification Ts ;
   - mesurer la force électromotrice E2 qui correspond à la teneur en élément soluté à mesurer dans le métal fondu, au moyen d'un électrolyte solide (26) qui présente une conductivité vis-à-vis des ions oxygène et qui est revêtu d'un oxyde qui contient l'élément soluté M à mesurer ; et
   - obtenir l'activité $a_M$ de l'élément soluté M à mesurer en se basant sur la force électromotrice mesurée E2, sur la concentration en carbone [C] et sur la température mesurée.

7. Procédé de mesure selon la revendication 6, caractérisé en ce que ledit élément soluté M à mesurer est du manganèse.

8. Procédé de mesure selon la revendication 6, caractérisé en ce que ledit élément soluté M à mesurer est du silicium.

9. Procédé de mesure selon la revendication 6, caractérisé en ce que ledit élément soluté M à mesurer est du phosphore.

10. Procédé de mesure selon la revendication 6, caractérisé en ce que ledit élément soluté M à mesurer est du chrome.

11. Sonde pour mesurer l'activité d'un élément soluté M dans du métal fondu, comprenant :
    - un corps de sonde (11, 13) pour la mesure de l'élément soluté ; et
    - un fil provenant d'une électrode de travail (14) et connecté électriquement au corps de détecteur (11, 13), et constituant un circuit de mesure d'une force électromotrice,
    - ledit corps de détecteur (11, 13) pour la mesure de l'élément soluté comprenant un électrolyte solide qui entoure une référence (25) et qui présente une conductivité vis-à-vis des ions oxygène, et une couche de revêtement (27) composée d'un oxyde qui contient un élément soluté M à mesurer, ou un oxyde composite de celui-ci, et couvrant la périphérie extérieure de l'électrolyte solide (26),
    - ledit oxyde ou ledit oxyde composite pour ladite couche de revêtement (27) ayant une composition telle qu'il est prévu deux parties d'équilibrage ou plus pour une force électromotrice E1, E2, mesurée par le circuit de mesure de la force électromotrice,
    - la force électromotrice E1, E2 étant définie par l'activité $a_M$ de l'élément soluté M à mesurer dans la première des parties d'équilibrage, et par l'activité de l'oxygène $a_O$ dans le métal fondu dans la dernière partie d'équilibrage.

12. Sonde de mesure selon la revendication 11, comprenant en outre un thermocouple (15, 16) pour mesurer la température T du métal fondu.

13. Sonde de mesure selon la revendication 11, caractérisée en ce que ladite couche de revêtement (27) dudit corps de détecteur (11, 13) pour la mesure de l'élément soluté contient de l'oxyde de manganèse ou un oxyde composite de celui-ci.

14. Sonde de mesure selon la revendication 13, caractérisée en ce que l'épaisseur de ladite couche de revêtement (27) est dans la plage de 30 à 300$\mu$m.

15. Sonde de mesure selon la revendication 11, caractérisée en ce que ladite couche de revêtement (27) dudit corps de détecteur (11, 13) pour la mesure de l'élément soluté contient de l'oxyde de silicium ou un oxyde composite de celui-ci.

16. Sonde de mesure selon la revendication 15, caractérisée en ce que l'épaisseur de ladite couche de revêtement (27) est dans la plage de 30 à 300$\mu$m.

**17.** Sonde de mesure selon la revendication 11, caractérisée en ce que ladite couche de revêtement (27) dudit corps de détecteur (11, 13) pour la mesure de l'élément soluté contient de l'oxyde de phosphore ou un oxyde composite de celui-ci.

**18.** Sonde de mesure selon la revendication 17, caractérisée en ce que l'épaisseur de ladite couche de revêtement (27) est dans la plage de 30 à 300$\mu$m.

**19.** Sonde de mesure selon la revendication 11, caractérisée en ce que ladite couche de revêtement (27) dudit corps de détecteur (11, 13) pour la mesure de l'élément soluté contient de l'oxyde de chrome ou un oxyde composite de celui-ci.

**20.** Sonde de mesure selon la revendication 19, caractérisée en ce que l'épaisseur de ladite couche de revêtement est dans la plage de 30 à 300$\mu$m.

**21.** Appareil pour mesurer l'activité d'un élément soluté M dans du métal fondu, comprenant :
- des moyens de mesure de température (15, 16) pour mesurer la température du métal fondu, et
- des moyens formant sonde (10, 30, 50) pour mesurer une force électromotrice E1 qui correspond à une teneur en oxygène et une force électromotrice E2 qui correspond à la teneur en élément soluté du métal fondu,
- lesdits moyens formant sonde (10, 30, 50) comprenant un corps de détecteur (11, 13) pour la mesure de l'élément soluté, et un fil menant depuis une électrode de travail (14) et connecté électriquement au corps de détecteur (11, 13) et constituant un circuit de mesure de la force électromotrice, ledit corps de détecteur (11, 13) pour la mesure de l'élément soluté comprenant un électrolyte solide (26) qui entoure un matériau de référence (25) et qui présente une conductivité vis-à-vis des ions oxygène, et une couche de revêtement (27) composée d'un oxyde qui contient un élément soluté M à mesurer, ou un oxyde composite de celui-ci, et couvrant la périphérie extérieure de l'électrolyte solide (26), ledit oxyde ou ledit oxyde composite pour ladite couche de revêtement ayant une composition telle que deux parties d'équilibrage ou plus sont prévues pour une force électromotrice E1, E2 mesurée par le circuit de mesure de la force électromotrice, la force électromotrice E1, E2 étant définie par l'activité $a_M$ de l'élément soluté M à mesurer dans la première des parties d'équilibrage, et par l'activité en oxygène $a_O$ dans le métal fondu dans la dernière partie d'équilibrage.

F I G. 1

F I G.  2

F I G.  3

MEASURED ELECTROMOTIVE FORCE E

PROBE IMMERSION TIME

F I G.  4

FIG. 5

EP 0 358 168 B1

F I G. 6

F I G. 8

F I G. 7

F I G. 9

F I G. 10

F I G.  11

F I G. 12

F I G. 13

F I G. 14

F I G. 15

FIG. 16

FIG. 17